# EUROPEAN PATENT APPLICATION

(11) **EP 4 582 407 A1**
(43) Date of publication of application: **09.07.2025**
(21) Application number: 24150520.5
(22) Date of filing: 05.01.2024
(51) Int. Cl.: C07C 5/333, C07C 11/04, C07C 11/06, B01J 29/24

(54) **EFFICIENT NON-OXIDATIVE DEHYDROGENATION OF ALKANES TO OLEFINS USING TRANSITION-METAL-CONTAINING ZEOLITES**

(71) Applicant: PAUL SCHERRER INSTITUT, 5232 Villigen PSI (CH)
(72) Inventor: Sushkevich, Vitaly, 5200 Brugg (CH); Artsiusheuski, Mikalai A., 5232 Villigen (CH); van Bokhoven, Jeroen, 8044 Zürich (CH)
(74) Representative: Fischer, Michael

(57) **Abstract**

Dehydrogenation of light alkanes to olefins with high yield under mild reaction conditions remains a substantial challenge. The present invention proposes an innovative strategy for the low-temperature non-oxidative dehydrogenation of alkanes, in particular ethane and propane, leveraging copper(I)-containing zeolite, in particular mordenite zeolite, as the active material. The fundamental concept is the stoichiometric reaction of copper(I) sites hosted in the zeolite framework with gaseous alkane resulting in the formation of hydrogen and olefin adsorbed over copper(I) centers in the form of a stable n-complex. Complete conversion of copper(I) sites can be attained at 573 K with a selectivity of 100% towards olefins. The formation of a highly stable adsorption complex shifts the thermodynamic equilibrium of the dehydrogenation reaction giving the yield of olefin more than 500 times greater than the gas-phase dehydrogenation reaction thermodynamic limit. Valuable olefins can be released to gas phase upon contact of the reacted material with water at near-ambient temperature. The active material can be regenerated without any detectable deactivation, hence enabling continuous cyclic operation.

## Description

The present invention relates to a method for the non-oxidative dehydrogenation of alkanes, in particular short-chain alkanes, to olefins.

Alkenes, or olefins, are the cornerstone of the modern chemical industry being the building blocks of basic plastics and feedstock of valuable chemicals and fuels. Ethylene and propylene are produced in amounts exceeding 120 MTPA. They are the raw materials for the corresponding polymers and essential commodities, such as ethylene oxide, propylene oxide, acetaldehyde, and acrylonitrile.

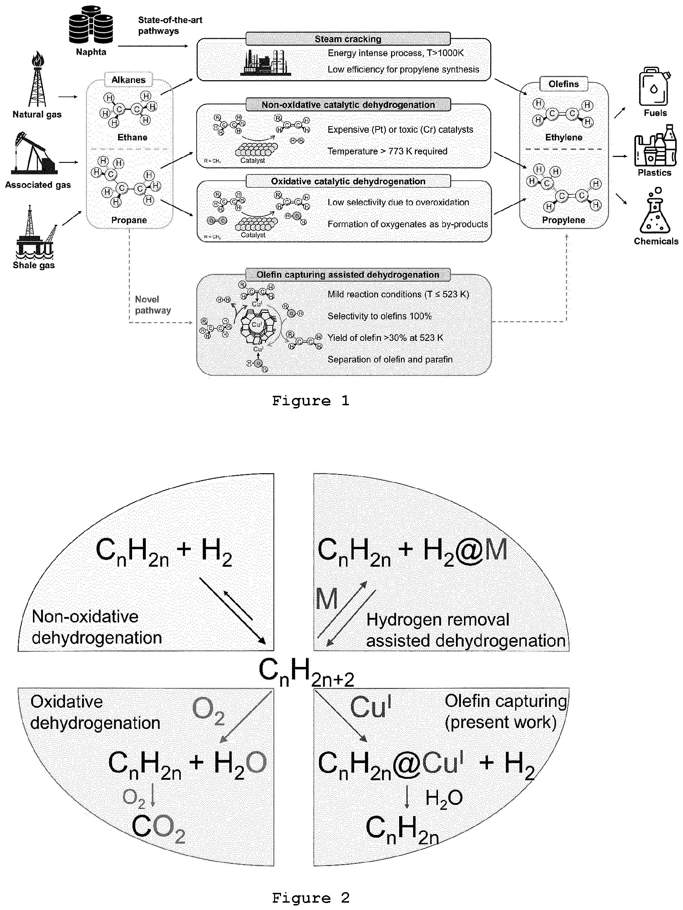

The primary method for the production of ethylene and propylene is steam cracking of naphtha or shale gas feedstock as shown in the upper process description in Figure 1. This process requires extremely high temperatures exceeding 1000 K, making it energy-inefficient and environmentally unfriendly due to significant carbon dioxide emission. Furthermore, the steam cracking has a much higher selectivity towards ethylene compared to higher olefins, and reorientation of steam cracking plants from naphtha to shale gas feedstock reduces the selectivity to C3+ products even more. This causes a shortage of propylene on the market, while the demand for it continuously increases, reaching 117 MTA in 2022, urging the elaboration of on-purpose technologies for propylene synthesis.

To fill the shortage, processes for the direct propane conversion to propylene via catalytic non-oxidative dehydrogenation (see second upper box in the middle of Figure 1) were developed and commercialized, with pioneers in the field being Lummus (Catofin technology) and UOP (Oleflex technology). Propane non-oxidative dehydrogenation processes demonstrate high selectivity towards propylene, above 85% for the aforementioned Catofin and Oleflex and above 90% for PDH (BASF-Linde). Despite being highly selective, non-oxidative dehydrogenation technologies have several drawbacks.

First, most processes currently utilize platinum- or chromium-containing catalysts, which are either costly or toxic. Even more importantly, all non-oxidative propane dehydrogenation technologies are operated at temperatures above 773 K due to thermodynamic constraints; for propane dehydrogenation at 723 K the equilibrium constant K= 8.3·10⁻³, limiting a theoretical propylene yield to 9% at 1 bar initial propane pressure. The modern catalysts enable operating close to the thermodynamic limit, and a better performance can be achieved only by altering the reaction thermodynamics. This cannot be accomplished by catalyst design, and novel concepts should be developed to overcome these thermodynamic limitations.

Several approaches aimed at decreasing the reaction temperature by shifting the equilibrium of dehydrogenation reaction have been suggested (Figure 2). A first strategy implies physical removal of the reaction by-product - hydrogen. This can be achieved by the utilization of selective hydrogen-permeable membrane reactors. This approach allows to increase the yield of olefin; however, the application of membranes is currently limited by their insufficient thermal stability and hydrogen permeability.

Alternatively, hydrogen can be removed from the reaction zone using hydrogen absorbers, or hydrogen scavengers, typically in a form of titanium or zirconium powders or liquid alloys of alkali metals prone to form stable hydrides. Despite this very straightforward concept, its capacity and efficiency are very limited due to diffusional constraints and necessity for regeneration of the material, complicating continuous operation.

Another much-researched way to avoid the thermodynamic limit is altering the reaction chemistry by carrying out oxidative dehydrogenation (third upper box in the middle of Figure 1). The concept implies adding an oxidant, typically oxygen, to reactants and formation of water instead of hydrogen, thus favorably shifting the thermodynamic equilibrium. An advantage compared to non-oxidative dehydrogenation is the possibility to operate at significantly lower temperature. However, the method is susceptible to unfavorable oxidation of the hydrocarbon molecules, leading to intrinsically low selectivity and formation of oxygenates as by-products with increasing conversion.

An alternative to the continuous catalytic oxidative dehydrogenation is chemical looping, where active material (copper-containing zeolite or ferric vanadate-vanadium oxide) acts as a stoichiometric oxidant and afterwards is regenerated with oxygen or air. While oxidative looping enables higher selectivity as compared to continuous catalytic processes, it is generally below 90% due to overoxidation. So far, no processes based on continuous or chemical looping oxidative propane dehydrogenation have been commercialized.

As discussed above, most approaches to shift the thermodynamic equilibrium target hydrogen removal, either physically (by utilization of membranes) or chemically (by scavengers or oxidants). Unfortunately, none of the proposed methods achieve the required efficiencies in the conversion process from alkanes to olefines.

Thus, it is the objective of the present invention to provide a method for the non-oxidative dehydrogenation of alkanes, in particular short-chain alkanes, to olefins that has a superior performance as compared to the methods currently known in the art and described above.

This objective is achieved according to the present invention by a method for the non-oxidative dehydrogenation of alkanes, in particular short-chain alkanes, to olefins, comprising the steps of:
a) bringing the alkane, preferably a pure alkane atmosphere or an atmosphere comprising the alkane diluted with an inert component, i.e. helium and/or nitrogen, in contact with a metal-comprising zeolite at an elevated temperature and ambient pressure or higher,
b) reacting the alkane with the metal cation within the zeolite thereby forming a metal-olefin complex within the zeolite and releasing gaseous hydrogen; and
c) bringing the zeolite comprising the metal-olefin complex in contact with water thereby exchanging the olefin ligand in the metal-olefin complex by water thereby releasing the olefin to the gas phase from the metal-olefin complex.

This method therefore allows to convert the alkanes into olefins at rather low temperature, due to the capturing properties of the metal cations hosted in zeolite for the copper-olefin complex, and with a high olefin yield and avoids the oxidation of the alkenes, because of the absence of an oxidant. The concept here is the reaction of the active sites hosted in the zeolite with gaseous alkane resulting in the formation of gaseous hydrogen and olefin adsorbed over metal centers in the form of a stable n-complex. The formation of a highly stable adsorption complex shifts the thermodynamic equilibrium of the dehydrogenation reaction giving a yield of olefin more than 500 times greater than the gas-phase dehydrogenation reaction thermodynamic limit. Valuable olefins can be released into the gas phase upon contact of the reacted material with water at near-ambient temperature, but also at a temperature where the alkane dehydrogenation reaction takes place or a temperature in between.

A preferred embodiment of the present invention can be achieved when the metal-water complex comprising zeolite framework is recycled into the method step a). Thus, the active zeolite material can be regenerated without any detectable deactivation, hence enabling continuous cyclic operation.

Typically, the elevated temperature may range from 100 to 300°C, preferably in the range from 150 to 250°C.

A further preferred embodiment of the present invention can be achieved when the release of the olefin from the zeolite can be supported by an inert atmosphere, preferably a helium and/or nitrogen atmosphere.

Advantageously, the zeolite can be selected from a group comprising MOR, MFI, FAU framework types and any other framework types that enable cation exchange.

Further advantageously, the metal, in cationic form in the zeolite can be selected from a group comprising copper, iron, nickel, and cobalt, in addition to any metal that in its cationic form forms complexes with olefins.

One preferred example with respect to the metal is copper being comprised in its copper(I) state and mordenite with respect to the zeolite.

Preferred embodiments of the present invention are described hereinafter with reference to the attached drawings which depict in:
- Figure 1: schematically three pathways according to the prior art and the pathway according to the present invention for the production of olefins from alkanes;
- Figure 2: a scheme of the present three methods according to the prior art and the method according to the present invention as shown in Figure 1 for on-purpose dehydrogenation of alkanes to olefins, wherein "M" in hydrogen removal assisted dehydrogenation refers to hydrogen scavenger material, such as metals (zirconium, titanium) or their alloys.
- Figure 3: schematic illustration of the reaction protocol (A) and results of ethane (B) and propane (C) dehydrogenation over Cu^{I}MOR in a plug-flow reactor at 573 K, dashed horizontal line corresponds to copper content in the material; and
- Figure 4: results of ethane (130 pmol) reaction with Cu^{I}MOR (copper amount equal to 430 pmol) in a batch reactor at 523 K for 24 h.

The method according to the present invention provides an alternative way to shift the thermodynamic dehydrogenation equilibrium by selectively capturing the target reaction product, olefin. Herein, for the first time, an innovative method for the low-temperature non-oxidative dehydrogenation of alkanes, in particular ethane and propane, implying olefin selective adsorption utilizing metal-containing zeolite, in particular copper(I)-containing mordenite, is presented. The metal sites in the zeolite both promote the dehydrogenation reaction and act as stoichiometric centers for capturing the olefin product through forming a stable n-complex. As one preferred embodiment of the present invention, copper(I) sites hosted in mordenite have been used to prove this innovative concept. The quantitative conversion of copper(I) centers can be achieved at 573 K with a gas-phase selectivity towards olefins of 100%. The formation of a strong adsorption complex has a profound impact on the thermodynamics of the non-oxidative dehydrogenation, enabling yields of ~ 500 times higher than what is achievable in gas-phase dehydrogenation reactions under the same conditions.

### Results and Discussion

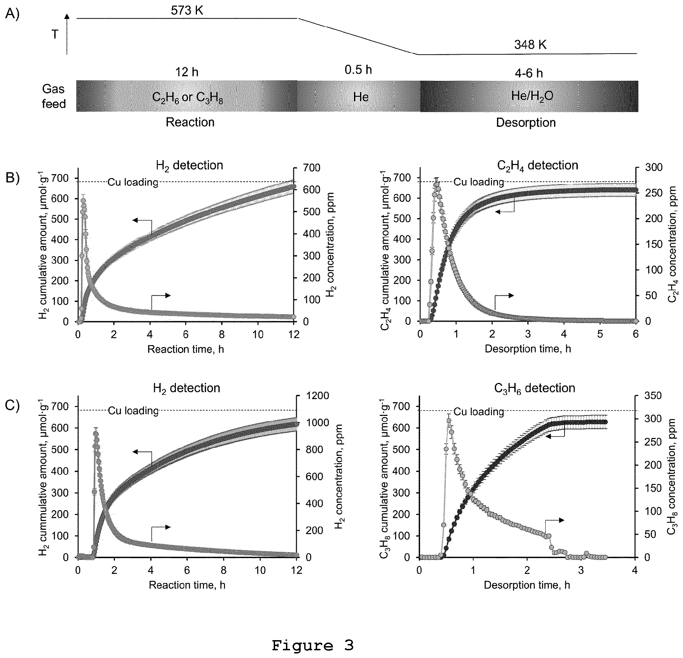

First, ethane and propane activation has been studied over copper(I)-containing mordenite (Cu^{I}MOR) in a plug-flow reactor. Cu^{I}MOR was prepared *in situ* from copper(II)-containing mordenite by reduction with methane at 673 K. Afterwards, the reaction between Cu^{I}MOR and ethane respectively propane was carried out at 573 K. The results of the dehydrogenation reaction are presented in Figure 3 and Table 1.

Figure 3 represents a schematic illustration of the reaction protocol (A) and results of ethane (B) and propane (C) dehydrogenation over Cu^{I}MOR in a plug-flow reactor at 573 K. Dashed horizontal line corresponds to the copper content in the zeolite material.

The contact of both ethane and propane with Cu^{I}MOR results in the formation of gaseous hydrogen as singular gas phase product. The subsequent exposure of the reacted material to a steam-containing helium flow leads to the exclusive release of olefins into the gas phase, and the cumulative amount of formed olefins closely matches the copper content in the material, as well as the cumulative amount of formed hydrogen (Figure 3D, Table 1). Notably, no products other than hydrogen were found in the outlet gas stream during the initial reaction and exclusively ethylene or propylene were detected during the steam-assisted desorption step.

For both ethane and propane conversion, the amounts of formed hydrogen and olefin match well the copper content in the Cu^{I}MOR material (Table 1). This indicates that i) selectivity in dehydrogenation is high and can reach up to 100% and ii) copper(I) centers act as stoichiometric reaction sites for the dehydrogenation reaction.

**Table 1. Results of ethane and propane dehydrogenation over Cu^{I}MOR in a plug-flow reactor at 573 K.**

| | Cu loading in material | C₂H₆ activation | | C₃H₈ activation | |
|---|---|---|---|---|---|
| | | H₂ released during reaction | C₂H₄ released during desorption | H₂ released during reaction | C₃H₆ released during desorption |
| Amount, µmol·g⁻¹ | 685±5 | 660±33 | 640±32 | 645±33 | 608±30 |
| Yield*, % | - | 96±5 | 93±5 | 94±5 | 89±5 |

| | | | | | |
|---|---|---|---|---|---|
| *Calculated as mol(product)/mol(Cu)*100% | | | | | |

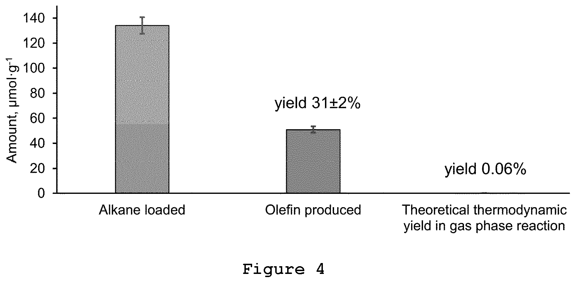

The dehydrogenation of ethane over Cu^{I}MOR was additionally studied in a batch reactor at 523 K. The results of the experiments are presented in Figure 4. Figure 4 shows the results of ethane (130 µmol) reaction with Cu^{I}MOR (copper amount equal to 430 pmol) in a batch reactor at 523 K for 24 h. The theoretical yield in gas-phase ethane dehydrogenation reaction is given for comparison. The amount of formed ethylene equals 5113 µmol, indicating that a conversion of ethane more that 30% can be achieved after 24 hours of reaction.

Importantly, this is enormously higher than the gas-phase thermodynamic limit. Under the selected experimental conditions, the change in standard Gibbs free energy for the gas-phase ethane dehydrogenation reaction equals ΔᵣG⁰= +73 kJ·mol⁻¹, resulting in K=5.1·10⁻⁸ and a theoretical olefin yield of 0.06%. The results of ethane dehydrogenation in the batch reactor demonstrate that the adsorption of the olefin shifts the thermodynamics of dehydrogenation reaction towards the formation of olefin, enabling dehydrogenation at mild reaction conditions with a yield significantly (500x times) surpassing the theoretical one of the gas-phase reaction.

**Table 2. Comparison of different approaches for propane dehydrogenation.**

| Type of propane dehydrogenation | Non-oxidatice catalytic | Non-oxidatice catalytic with H₂ membrane separation | Non-oxidatice catalytic with H2 scavenging | Oxidative catalytic | Olefin capturing assisted (present invention) |
|---|---|---|---|---|---|
| Operating temperature, K | > 773 | > 773 | > 773 | > 673 | 523 |
| Selectivity towards propylene, mol % | 85-90 | 80-90 | 85-90 | < 75 | 100 |
| Conversion of alkane, % | 30-40 | 30-50 | 35-50 | <15 | >30 |
| Propane/propylene separation | Required as separate step | Required as separate step | Required as separate step | Required as separate step | Implemente d in process |

In summary, the method according to the present invention with the concept of olefin scavenging dehydrogenation demonstrates - even without further optimization of conditions - several fundamental advantages compared to state-of-the-art dehydrogenation technologies (Table 2).

Remarkably, the method operates at temperature as low as 523 K with a conversion of alkane above 30%, while all other dehydrogenation processes require temperatures above 723 K to achieve similar conversion. This is enabled by manipulating the reaction thermodynamics via strong adsorption of the resulting olefin. Importantly, other methods focusing on shifting the equilibrium, i.e. hydrogen scavenging or its selective removal through membranes, still operate at above 723 K with a much lower productivity.

To the best of the present knowledge in the art, there are no successful examples showing the decrease in reaction temperature by 200 K by means of utilization of physical hydrogen removal. The alkane dehydrogenation can be performed at slightly lower temperature around 673 K via oxidative dehydrogenation, yet the selectivity towards olefin is significantly lower (Table 2). This demonstrates another advantage of olefin capturing: strong adsorption protects the target molecule from further transformation, enabling an extraordinary selectivity of 100%. Finally, in the method according to the present invention of the olefin scavenging-assisted dehydrogenation method, the costly alkane/olefin separation is not needed. Instead, the alkane and alkene evolution from the reactor are temporally separated. This is crucial for dehydrogenation and other petrochemical processes, as alternative to the state-of-the-art cryogenic distillation, which is an extremely energy-intensive process.

### Preferred examples

### Example 1

The commercial mordenite zeolite with Si/Al ratio of 6 in sodium form (CBV 10A, Zeolyst) was used as a starting material. Ion-exchange to copper was performed according to the following experimental procedure: 5 g of zeolite were added to 500 mL of 0.05 M solution of copper (II) nitrate (99+%, Sigma-Aldrich) and the suspension was stirring for 12 h at 323 K. Afterwards, the suspension was filtered through a paper porous filter, rinsed with deionized wared and dried at 393 K overnight. The procedure was repeated three times, and the resulting copper-containing mordenite material was as designated as "CuMOR".

200 mg of as prepared sample in a form of 0.25-0.5 mm fraction was put into quartz plug-flow reactor, heated in a flow of oxygen (40 ml·min⁻¹) to 723 K at 1 bar for 60 min with heating rate of 5 K·min⁻¹ for the activation. The activated sample was afterwards put in contact with flow of methane at 673 K for 1 h for the conversion of copper(II) species to copper(I) sites. Next, the sample was cooled to 573 K with cooling rate of 10 K·min⁻¹ in a flow of methane (Messer 4.8), and helium flow (55 ml·min⁻¹) was introduced for 10 minutes at the desired temperature. Afterwards, a flow of ethane (Messer 4.5, 50 ml·min⁻¹) was introduced for the reaction. After the reaction is complete, a gas flow was switched to helium (55 ml·min⁻¹) and the sample was cooled to 348 K. The products of partial oxidation were desorbed by interaction of sample with a wet stream of helium (55 ml·min⁻¹), prepared by bubbling helium through the saturator with liquid water kept at 298 K resulting in He/H₂O=40/1. After the complete desorption of products, the reactor was purged with dry helium (55 ml·min⁻¹) for 10 min and heated up to 723 K with the heating rate of 7 K·min⁻¹ in dry helium to verify that there are no other strongly adsorbed products. The amount of the reaction products is provided in Table 1.

### Example 2.

The process is carried out as described in the example 1 with propane used instead of ethane. Parameters of the process are given in Table 1.

### Example 3.

The reaction was performed in modified reaction setup enabling the contact of material with fixed amount of gas phase (batch reaction). For the experiment, 625 mg of CuMOR in a form of 0.25-0.5 mm fraction was placed into stainless-steel reactor, and the additional quartz rods were used as spacers to reduce the volume of the gas in the reactor. The volume of the reactor available for gas phase in the experiments corresponded to 20 mL. The activation procedure was identical to the described in example 1. After the activation, reactor was purged with the flow of helium (55 ml·min⁻¹) for 10 minutes, and the He/C₂H₆=7/1 flow with total rate of 70 ml·min⁻¹ was introduced. After keeping flow for 10 minutes, the inlet and outlet valves to the reactor were closed and the reactor containing the material and gas phase was heated up to 523 K and kept for 24 hours. After that, reactor was cooled down to 348 K, inlet and outlet valves were open and a flow of helium (55 ml·min⁻¹) was introduced for 10 minutes, followed by switch to wet stream of helium (55 ml·min⁻¹) for the desorption of the reaction products. The process parameters are given in the Figure 3 and text above.

## Claims

1. A method for the non-oxidative dehydrogenation of alkanes, in particular short-chain alkanes, to olefins, comprising the steps of:
a) bringing the alkane, preferably a pure alkane atmosphere or an atmosphere comprising the alkane diluted with an inert component, i.e. helium and/or nitrogen, in contact with a metal-comprising zeolite at an elevated temperature and ambient pressure or higher,
b) reacting the alkane with the metal cation within the zeolite thereby forming a metal-olefin complex within the zeolite framework and releasing gaseous hydrogen; and
c) bringing the zeolite framework comprising the metal-olefin complex in contact with water thereby exchanging the olefin ligand in the metal-olefin complex by water thereby releasing the olefin to the gas phase from the metal-olefin complex.

2. The method according to claim 1 wherein the metal-water complex comprising zeolite is recycled into the method step a) .

3. The method according to claim 1 wherein the elevated temperature ranges from 100 to 300°C, preferably in the range from 150 to 250°C.

4. The method according to any of the preceding claims wherein the release of the olefin from the zeolite is supported by an inert atmosphere, preferably a Helium and/or a nitrogen atmosphere.

5. The method according to any of the preceding claims wherein the zeolite is selected from a group comprising MOR, MFI, FAU, and any other zeolite framework types that enable cation exchange.

6. The method according to any of the preceding claims wherein the metal in the zeolite framework is selected from a group comprising copper, iron, nickel, cobalt, in addition to any metal that in its cationic form forms complexes with olefins.

7. The method according to claim 6 wherein the copper and/or any other metal is comprised in its copper(I), respectively in its cationic state.
